# EUROPÄISCHE PATENTSCHRIFT

(11) **EP 0 551 052 B1**
(45) Veröffentlichungstag und Bekanntmachung des Hinweises auf die Patenterteilung: **01.05.1996**
(21) Anmeldenummer: 92810939.6
(22) Anmeldetag: 02.12.1992
(51) Int. Cl.: C07C 205/06, C07C 201/08, B01J 29/18

(54) **Verfahren zur Gasphasennitrierung von Aromaten mittels auf H-Mordenit basierenden Katalysatoren**
Process for the gas phase nitration of aromates with H-mordenite based catalysts
Procédé pour la nitration de composés aromatiques en phase gazeuse à l'aide de catalyseurs à base de H-mordenite

(30) Priorität: 04.12.1991 CH 3554/91
(43) Veröffentlichungstag der Anmeldung: 14.07.1993
(73) Patentinhaber: CU CHEMIE UETIKON AG, CH-8707 Uetikon am See (CH)
(72) Erfinder: Kouwenhoven, Herman W., CH-8704 Herrliberg (CH); Bertea, Leopoldo, CH-8052 Zürich (CH); Prins, Roel, CH-8032 Zürich (CH)
(74) Vertreter: Werffeli, Heinz R., Dipl.-Ing.ETH.

(56) Entgegenhaltungen:
- EP-A- 0 053 031
- GB-A- 2 000 141
- PATENT ABSTRACTS OF JAPAN vol. 13, no. 21 18. Januar 1989 & JP-A-63 225 339 (RES ASSOC UTIL OF LIGHT OIL) 20. September 1988
- TETRAHEDRON LETTERS Bd. 30, Nr. 39, 1989, OXFORD GB Seiten 5333 - 5336 K. SMITH ET AL. 'Para-selective mononitration of alkylbenzenes under mild conditions by use of benzoyl nitrate in the presence of a zeolite catalyst'
- ET AL. 'Para-selective mononitration of alkylbenzenes under mild conditions by use of benzoyl nitrate in the presence of a zeolite catalyst'

## Beschreibung

Gegenstand der Erfindung ist ein Verfahren zur Herstellung von Nitrobenzol durch Gasphasennitrierung von Benzol mit verdünnter oder konzentrierter Salpetersäure mit einem Salpetersäure Gehalt von 15 bis 95 Gew%, vorzugsweise von mehr als 50 Gew%, in Gegenwart von auf H-Mordenit basierenden Katalysatoren.

Verfahren zur Gasphasennitrierung von Aromaten, in denen NO₂ oder andere Stickoxide als Nitriermittel angewandt werden, sind schon mehrfach in der einschlägigen Literatur beschrieben worden. Eine Übersicht in US-Pat. 4.754.083 zeigt, dass die Anwendung einer grossen Anzahl von unterschiedlichen Materialien als Katalysatoren für die Gasphasennitrierung von Aromaten mit Stickoxyden beansprucht wurde. Zeolithe und andere kristalline und amorphe Aluminosilikate werden in dieser Übersicht besonders häufig als Katalysatoren erwähnt. In US-Pat. 4.754.083 werden Molekularsiebe, Montmorillonit und mittels sog. Pfeilern ausgeweiteter Bentonit im Allgemeinen als Katalysatoren für die Gasphasennitrierung von substituierten Aromaten, welche eine meta-dirigierende Gruppe wie -NO₂ enthalten, beansprucht. Eine grosse Anzahl von unterschiedlichen Zeolithen, unter andern Zeolon 900H, H-Y, H-Beta, H-ZSM5, Na-X, H-Ferrierit und H-Erionit werden im US-Pat. 4.754.083 als Katalysatoren für die Gasphasennitrierung von Nitrobenzol angewandt. In der Beschreibung im US-Pat. 4.754.083 der durchgeführten Experimente wird die Anwendung von zwei verschiedenen Reaktoren erwähnt. Ergebnisse über Umsatz, gemessen in beiden Reaktortypen nach eins bis zwei Stunden Reaktionsdauer, werden beschrieben. Diese Daten sind somit nicht ausreichend, um Aussagen über die Langzeitstabilität der Katalysator-Leistung machen zu können. Die bevorzugten Katalysatoren, basierend auf Zeolith Na-X oder H-Ferrierit, zeigen einen niedrigen Umsatz (<25%) bei einer erhöhten Selektivität für p-Dinitrobenzol. In der EP-A-0 343 048 wird ein Gasphasenverfahren zur Herstellung von Nitrobenzol mit Salpetersaure vorgeschlagen, in der Katalysatoren basierend auf sauren Schichtsilikaten oder sauren oxidischen Materialien angewandt werden, die Oxide aus der Gruppe IVA des periodischen Systems zusammen mit Wolframtrioxid, Molybdäntrioxid oder Zinkoxid umfassen. Stabilität, Aktivität und Selektivität der in der EP-A-0 343 048 erwähnten bevorzugten Katalysatoren sollen höher sein als bei den früher beschriebenen Katalysatoren.
Die Anwendung von auf H-Mordenit basierenden Katalysatoren in der Gasphasennitrierung von Aromaten mittels NO₂, N₂O₄, oder N₂O₃, vorzugsweise mit NO₂, wird in einer Reihe von Patenten, die u.a. EP-A-053 053; EP-A-078 247; EP-A-092 372 und US-Patent 4.107.220 umfasst, beschrieben. Auf H-Mordenit basierende Katalysatoren, die in diesen Patenten erwähnt werden, sind Handelsprodukte und wurden durch die Norton Cy als Zeolon-xxxH (z.B. Zeolon 200H oder Zeolon 900H) vermarktet. Ausser einer Vorbehandlung mit dem bevorzugten Nitriermittel bei Reaktionsbedingungen wird dort keine weitere spezifische Vorbehandlung als wichtig für die Katalysatorleistung angesehen. In der DE-OS 2,826,433 und im US-Patent 4.418.230, beide von J. M. Bakke und J. Liaskar, wird Zeolon 200H als Katalysator für die Gasphasennitrierung von Toluol mit Salpetersäure angewandt. Auch in diesen Patenten wird keine weitere Vorbehandlung des Zeolithen als wichtig für die Katalysatorleistung genannt. Die Versuche wurden bei Normaldruck und 473K und einem Molverhältnis von Aromat und Salpetersäure von etwa 1,4 durchgeführt. Daten über die Stabilität der Katalysatorleistung werden nicht gegeben. Die Leistung von dem auf Mordenit basierenden Katalysator ist niedrig im Vergleich mit der eines auf Montmorillonit basierenden Katalysators, und die meisten Daten beziehen sich auf Katalysatoren auf Basis Montmorillonit.

Mordenit ist ein Molekularsieb, der als Mineral in der Erdkruste vorkommt und auch synthetisch hergestellt werden kann. Die allgemeine Zusammensetzung von Mordeniten ist Na₈[Al₈Si₄₀O₉₆].24H₂O, die Struktur von Mordenit wird beschrieben im "Atlas of Zeolitic Structure Types" von W.M. Meier und D.H. Olson, zweite Ausgabe, im Auftrag von der "International Zeolite Association" in 1987 vom Verlag Butterworth herausgegeben. Die negative elektrische Ladung des Mordenit Gitters, verursacht durch die Substitution von Silizium- durch Aluminium-Atome, wird durch die austauschbaren Natriumionen ausgeglichen. In vielen Anwendungen der Katalyse werden saure Zeolithe, öfters als H-Zeolithe bezeichnet, benutzt, die mittels Ionenaustausch-Verfahren hergestellt werden. H-Mordenit wird entweder direkt durch Ionenaustausch mit Säuren oder indirekt durch eine Kalzinierung von Ammonium-Mordenit hergestellt. Die Methode zur Herstellung des H-Mordenits übt öfters einen entscheidenden Einfluss aus auf die Katalysatorleistung des gebildeten Materials in einer katalytischen Reaktion. Diese Beinflussung wird zum Beispiel in der Anwendung von auf Mordenit basierenden Katalysatoren in der Alkylierung von Biaromaten im US-Patent 4,891,448 sowie in der EP-A 0 317 907, beide von C.S. Lee et al., und in der Anwendung von auf Mordenit basierenden Katalysatoren in der Isomerisierung von Paraffinen in der DE-OS 1,816,822 von H. J. A. van Helden und H.W.Kouwenhoven, deutlich gezeigt. Aus diesen oben erwähnten Veröffentlichungen kann man erschliessen, dass keine eindeutige Methode zur Herstellung von einem optimalen auf H-Mordenit basierenden Katalysator für Anwendungen in unterschiedlichen Reaktionen definiert werden kann, und dass die optimale Herstellungsmethode für jede Anwendung von auf H-Mordenit basierenden Katalysatoren untersuchenswert ist.

Die Gasphasennitrierung von Benzol gemäss der vorliegenden Erfindung wird mit Katalysatoren basierend auf Mordenit durchgeführt. Die Katalysatoren werden durch Anwendung von Ionenaustauschverfahren und Kalzinierungen aus Na-Mordenit hergestellt. Zufolge dieses Herstellungsverfahrens werden erstens Natriumionen teilweise oder ganz entfernt und ersetzt durch Protonen, und zweitens werden Aluminiumatome aus deren herkömmlichen Gitterstellen, in der Fachliteratur mit T-Sites angedeutet, entfernt und ersetzt durch Siliziumatome. Das so gebildete Oxoaluminium Material stellt eine separate Phase neben dem Mordenit dar und kann normalerweise durch Extrahieren mittels Säuren entfernt werden. Es wird vorgeschlagen die Gasphasennitrierung von Benzol bei einer Temperatur zwischen 423 K und 523 K, mit verdünnter oder konzentrierter Salpetersäure mit einem Salpetersäure Gehalt von 15 bis 95 Gew%, vorzugsweise von mehr als 50 Gew%, in Gegenwart von auf Mordenit basierenden Katalysatoren durchzuführen. Es wurde überraschend gefunden dass die Katalysatorleistung des H-Mordeniten in der Gasphasennitrierung von Benzol durch Kalzinierung bei Temperaturen höher als 823 K stark beeinträchtigt und durch eine darauffolgende Säurebehandlung stark verbessert wird.

Die Erfindung wird anschliessend anhand von Ausführungsbeispielen erläutert. Die Katalysatorherstellung wird in den Bei-spielen 1 bis 5 beschrieben. Die Prüfung der so hergestellten Katalysatoren und von Norton 900H wird in den Beispielen 10 bis 18, und die Prüfung von Vergleichskatalysatoren wird in den Beispielen 6 bis 9 beschrieben.

### Herstellung der Katalysatoren

### Beispiel 1.

Bei der Herstellung der Katalysatoren wurde handelsüblicher Mordenit, hergestellt von der CU Chemische Fabrik Uetikon, Typ PM-1Na, verwendet. Die analytischen Daten sind in der Tabelle 1 erwähnt. Dieses Material wurde wie folgt aktiviert: 500 g PM-1Na wurden in 5000 ml einer 1M Lösung von Salzsäure aufgeschlämmt und während einer Stunde unter Rühren bei 373 K erhitzt. Die Aufschlämmung wurde anschliessend filtriert und mit 5000 ml demineralisiertem Wasser gewaschen. Der während einer Stunde bei 373 K getrocknete Filterkuchen bildet Probe A. 50 g von Probe A wurden in 500 ml von einer ein molaren wässrigen Ammoniumnitrat Lösung aufgeschlämmt und während einer Stunde unter Rühren bei 373 K geheizt. Die Aufschlämmung wurde filtriert und mit 500 ml demineralisiertem Wasser gewaschen. Das Produkt wurde noch einmal in 500 ml einer ein molaren wässrigen Ammoniumnitrat Lösung aufgeschlämmt und während einer Stunde unter Rühren bei 373 K geheizt. Die Aufschlämmung wurde filtriert und mit 500 ml demineralisiertem Wasser gewaschen. Ein Teil des Filterkuchens wurde während 16 Stunden bei 393 K getrocknet und während drei Stunden bei einer Temperatur von 773 K kalziniert und bildet Probe B. Ein anderer Teil des Filterkuchens wurde in einem geschlossenen Tiegel während fünf Stunden bei 823 K kalziniert und bildet Probe C. Ein dritter Teil des Filterkuchens wurde in einem geschlossenen Tiegel während fünf Stunden auf 973 K kalziniert und bildet Probe D. Die analytischen Daten werden in der Tabelle 1 erwähnt.

### Beispiel 2.

200 g von Probe A wurden in 1000 ml einer ein molaren Lösung von Salzsäure aufgeschlämmt und während einer Stunde unter Rühren bei 373 K geheizt. Die Aufschlämmung wurde filtriert und dreimal mit 500 ml demineralisiertem Wasser gewaschen. Das Material wurde noch einmal in 1000 ml von einer ein molaren Lösung von Salzsäure aufgeschlämmt und während einer Stunde unter Rühren bei 373 K geheizt. Die Aufschlämmung wurde filtriert und und dreimal mit 500 ml demineralisiertem Wasser gewaschen. Der während 16 Stunden bei 393 K getrocknete Filterkuchen bildet Probe E. Die analytischen Daten sind in der Tabelle 1 erwähnt.

### Beispiel 3.

150 g von Probe E wurden während drei Stunden in Luft bei 923 K kalziniert. Das Produkt bildet Probe F. 120 g von Probe F wurden in 1000 ml sechs molarer Salzsäure aufgeschlämmt und 16 Stunden unter Rühren bei 381 K erhitzt. Die Aufschlämmung wurde filtriert und dreimal mit 500 ml demineralisiertem Wasser gewaschen. Der während 24 Stunden bei 393 K getrocknete Filterkuchen bildet Probe G. Die analytischen Daten sind in der Tabelle 1 erwähnt.

### Beispiel 4.

100 g von Probe G wurden während drei Stunden in Luft bei 923 K kalziniert. Das Produkt bildet Probe H. 50 g von Probe H wurden in 500 ml sechs molarer Salzsäure aufgeschlämmt und 16 Stunden bei 381 K unter Rühren erhitzt. Die Aufschlämmung wurde filtriert und dreimal mit 500 ml demineralisiertem Wasser gewaschen. Der während 24 Stunden bei 393 K getrocknete Filterkuchen bildet Probe J. Die analytischen Daten sind in Tabelle 1 erwähnt.

**Tabelle 1**

| Probe | SiO₂/Al₂O₃ | Na₂O | Oberfläche,m²/g* Volumen ml/g* | | |
|---|---|---|---|---|---|
| | | | Total | Aeussere | Mikroporen |
| 900H | 10 | 0.75 | 460 | 59 | 0,17 |
| PM1-Na | 12,4 | 7,16 | 168 | - | - |
| B | 12,5 | <0,01 | 480 | 61 | 0,20 |
| C | 12,5 | <0,01 | 490 | 60 | 0,20 |
| D | 12,5 | <0,01 | 495 | 87 | 0,19 |
| E | 13,2 | 0,42 | 495 | 72 | 0,20 |
| F | 13,2 | 0,42 | 510 | 76 | 0,20 |
| G | 108 | <0,01 | 486 | 120 | 0,17 |
| H | 108 | <0,01 | 502 | 111 | 0,18 |
| J | 117 | <0,01 | 485 | 113 | 0,18 |
| K | 40 | <0,01 | 509 | 119 | 0,18 |
| L | 44,2 | <0,01 | 499 | 121 | 0,18 |

| | | | | | |
|---|---|---|---|---|---|
| * Gemessen mit Ar in einem Micromeritics ASAP 2000M nach Standardverfahren. | | | | | |

### Beispiel 5.

50 g von Probe E wurden in 500 ml einer sechs molaren Lösung von Salzsäure in Wasser aufgeschlämmt und unter Rühren während 16 Stunden bei 381 K erhitzt. Die Aufschlämmung wurde filtriert und dreimal mit 500 ml demineralisiertem Wasser gewaschen. Der während 24 Stunden bei 393 K getrocknete Filterkuchen bildet Probe K. 25 g von Probe K wurden in 500 ml sechs molarer Salzsäure aufgeschlämmt und unter Rühren wahrend 16 Stunden bei 381 K erhitzt. Die Aufschlämmung wurde filtriert und dreimal mit 500 ml demineralisiertem Wasser gewaschen. Der während 24 Stunden bei 393 K getrocknete Filterkuchen bildet Probe L. Die analytischen Daten sind in der Tabelle 1 erwähnt.

### Katalysatorprüfung

Nach einem ersten Verfahren wurde die Gasphasennitrierung von Benzol in kontinuierlichen Experimenten während mindestens 3 Stunden in einem Festbettreaktor durchgeführt. Die folgenden Reaktionsbedingungen wurden für den Vergleich der Katalysatoren gewählt:

| | |
|---|---|
| Raumgeschwindigkeit Benzol | 1 kg/kg(Kat)Stunde |
| Raumgeschwindigkeit Salpetersäure | 4 kg/kg(Kat)Stunde |
| Temperatur | 443 K |
| Druck | atmosph. Druck |

In den folgenden Versuchen wurde käufliche wässrige 65%Gew Salpetersäure benützt; das angegebene Molverhältnis Benzol/Salpetersäure bezieht sich auf 100% Salpetersäure. Das Trägergas war ein 1:1 Luft/Stickstoff Gemisch.

Nach einem zweiten Verfahren wurde die Gasphasennitrierung von Benzol in kontinuierlichen Experimenten während mindestens 20 Stunden in einem Festbettreaktor durchgeführt. Die folgenden Reaktionsbedingungen wurden für den Vergleich der auf Mordenit basierenden Katalysatoren gewählt:

| | |
|---|---|
| Raumgeschwindigkeit Benzol | 1 kg/kg(Kat) Stunde |
| Raumgeschwindigkeit Salpetersäure | 0.4 kg/kg(Kat).Stunde |
| Temperatur | 443 K |
| Druck | atmosph. Druck |

In den folgenden Versuchen wurde käufliche wässrige 65%Gew Salpetersäure benützt; das angegebene Molverhältnis Benzol/ Salpetersäure bezieht sich auf 100% Salpetersäure. Das Trägergas war entweder ein 1:1 Luft/Stickstoff Gemisch, oder reiner Stickstoff. Das Volumen Verhältnis Trägergas/Benzol (Dampf) war etwa 1,4. In den Versuchen nach dem zweiten Verfahren waren die Partialdrücke (in mbar) der verschiedenen Komponenten im Eduktstrom : Salpetersäure 138; Benzol 281; Wasser 256; Luft/Stickstoff 385.

Die Katalysatoren wurden vor Gebrauch in einer Druckpresse pelletiert und zu einer Körnung von 0.3 07 mm zerkleinert. Sie wurden entweder nach einer Kalzinierung bei einer Temperatur von 773 K und einer anschliessenden Aequilibrierung bei Raumtemperatur in Luft oder aber ohne vorangehende Kalzinierung im Reaktor eingesetzt und dort bei Reak-tionstemperatur und Trägergasdurchfluss während mindestens zweier Stunden vorbehandelt. Daraufhin wurde der Katalysator während 30 min bei Reaktionsbedingungen mit Salpetersäuredämpfen vorbehandelt; während dieser Behandlung wurde meistens eine Temperatursteigerung beobachtet. Die optimale Zeitdauer der Behandlung mit Salpetersäuredämpfen hängt natürlich von der eingesetzten Menge Katalysator und von den gewählten Reaktionsbedingungen ab und wird vorzugsweise betrieben, bis der Katalysator mit Salpetersäuredämpfen gesättigt ist. Die hier angegebene Behandlungsdauer von 30 min ist also nur als Beispiel zu betrachten. Nach Erreichen des Temperaturgleichgewichts wurde verdampftes Benzol zugemischt, auch dann wurde meistens ein exothermes Verhalten beobachtet, das durch den zeitlich bedingten Salpetersäure-Überschuss auf dem Katalysator verursacht wurde. Die Reaktionsprodukte wurden nach einer Einlaufperiode von einer Stunde in einer mit Aceton gefüllten Kühlfalle bei 278K gesammelt, und periodisch mittels GC in Bezug auf die organischen Anteile analysiert. Die unverbrauchte Salpetersäure wurde durch Titration ermittelt.

### Vergleichsbeispiele 6-9:

Das erste Verfahren zur Katalysatorprüfung laut obiger Beschreibung wurde für folgende Materialien angewandt. Beispiel 6: Glaswolle; Beispiel 7: amorphes Siliziumdioxid; Beispiel 8: Quartz; Beispiel 9: Norton Zeolon 900H. Tabelle 2 enthält die Resultate, die in den unterschiedlichen Prüfungsverfahren erhalten wurden.

**Tabelle 2**

| Vergleichsbeispiele 6-9. | |
|---|---|
| Beispiel Nr. | Ausbeute bezüglich Benzol in % (Zeit,Stunden) |
| 6 | 2(2) |
| 7 | 3(2) |
| 8 | 12(2) |
| 9 | 29(2) |

### Beispiele 10-18

Das zweite Verfahren zur Katalysatorprüfung nach obiger Beschreibung wurde für folgenden Materialien angewandt. Beispiel 10: Zeolon 900H; Beispiel 11: Probe B; Beispiel 12: Probe C; Beispiel 13: Probe D; Beispiel 14; Probe E; Beispiel 15: Probe F; Beispiel 16: Probe H; Beispiel 17: Probe J; Beispiel 18: Probe L.

Tabelle 3 beinhaltet die Resultate, die in den unterschiedlichen experimentellen Beispielen 10 bis 18 erhalten wurden. Die Selektivität bezüglich Benzol war in all diesen Experimenten besser als 99.9%. Alle Experimente wurden während mindestens 20 Stunden durchgeführt.

**Tabelle 3**

| Beispiele 10-18. | | | | | | | | | |
|---|---|---|---|---|---|---|---|---|---|
| | Beispiel | | | | | | | | |
| | 10 | 11 | 12 | 13 | 14 | 15 | 16 | 17 | 18 |
| Ausbeute Nitrobenzol | | | | | | | | | |
| bezüglich HNO₃(%)* | 45/20 | 69 | 70 | 65/36 | 64 | 70/63 | 75 | 76 | 69 |
| Umsatz Benzol(%) | 23/10 | 32 | 34 | 35/18 | 33 | 34/31 | 34 | 35 | 34 |

| | | | | | | | | | |
|---|---|---|---|---|---|---|---|---|---|
| * Falls Desaktivierung vorkam, werden zwei Werte genannt, wobei sich der erste auf das Resultat nach zwei Stunden und das zweite sich auf das Resultat nach 20 Stunden bezieht. | | | | | | | | | |

Die Daten in Tabelle 2 zeigen, dass Glaswolle und Quartz keine katalytische Aktivität für die Gasphasennitrierung von Benzol mittels verdünnter Salpetersäure aufwiesen. Amorphes Siliziumdioxid und Norton Zeolon 900H zeigten eine mittlere Aktivität und eine niedrige Stabilität. Norton Zeolon 900H, für welches ein SiO₂/Al₂O₃ Verhältnis von 10 angegeben wird, ist nach den EP-A-0 053.053, EP-A-0 078.247, EP-A-0 092.372 und dem US-Patent 4.107 220 als ein bevorzugter Katalysator für die Nitrierung von Aromaten mittels Stickstoffdioxid erwähnt. In der DE-OS 2,826,433 und im US-Patent 4.418.230, beide von J.M.Bakke und J.Liaskar, wird das Zeolon 200H, für welches ein SiO2/Al2O3 Verhältnis von 10 angegeben wird, als Katalysator für die Gasphasennitrierung von Toluol mit Salpetersäure vorgeschla-gen. Die Daten in Tabelle 3 zeigen, dass bei der Gaspha-sennitrierung von Benzol die Leistung von Katalysatoren auf Basis von H-Mordenit stark durch deren Herstellungsmethode beeinflusst wird und dass Norton 900H im Vergleich mit den anderen auf Mordenit basierenden Katalysatoren eine niedrige Aktivität und Stabilität zeigt.

## Patentansprüche

1. Verfahren zur Herstellung von Nitrobenzol dadurch gekennzeichnet, dass man Benzol bei einer Temperatur zwischen 400 und 523 K mittels wässriger Salpetersäure in der Gasphase nitriert, in Gegenwart von auf H-Mordenit basierenden Katalysatoren, die ein SiO₂/Al₂O₃ Verhältnis grösser als 12 besitzen und weniger als 0.5Gew% Na₂O enthalten.

2. Verfahren nach Anspruch 1, dadurch gekennzeichnet dass die Gasphasennitrierung mit wässriger Salpetersäure mit einer Konzentration höher als 50Gew% durchgeführt wird.

3. Verfahren nach den Ansprüchen 1 bis 2, dadurch gekennzeichnet, dass die Katalysatoren ein SiO₂/Al₂O₃ Verhältnis grösser als 40 besitzen.

4. Verfahren nach den Ansprüchen 1 bis 3, dadurch gekennzeichnet, dass die Katalysatoren ein SiO₂/Al₂O₃ Verhältnis grösser als 100 besitzen.

5. Verfahren nach den Ansprüchen 1 bis 4, dadurch gekennzeichnet, dass der H-Mordenit Zeolith Katalysator weniger als 0.1 Gew% Na₂O enthält.

## Claims

1. A process for the preparation of nitrobenzene, characterized by the gasphase nitration of benzene at a temperature between 400 and 523K using aqueous nitric acid in the presence of catalysts based on H-mordenite, having a SiO₂/Al₂O₃ molar ratio higher than 12 and a Na₂O content lower than O,5 wt%.

2. A process according to claim 1, wherein aqueous nitric acid having a nitric acid concentration above 50 wt% is used as nitrating agent in the gasphase nitration.

3. A process according to the claims 1-2, wherein the catalysts have a SiO₂/Al₂O₃ molar ratio higher than 40.

4. A process according to the claims 1-3, wherein the catalysts have a SiO₂/Al₂O₃ molar ratio higher than 100.

5. A process according to claims 1-4, characterized by that the H-mordenite zeolite catalyst has a Na₂O content lower than O,1wt%.

## Revendications

1. Procédé pour la préparation de nitrobenzène, caractérisé en ce qu'on procède à la nitration du benzène à une température entre 400 et 523 K au moyen d'acide nitrique aqueux en phase gazeuse, en présence de catalyseurs à base de H-Mordénite qui possèdent un rapport SiO₂/Al₂O₃ supérieur à 12 et qui contiennent moins de 0,5% en poids de Na₂O.

2. Procédé selon la revendication 1, caractérisé en ce que la nitration en phase gazeuse est réalisée avec de l'acide nitrique aqueux à une concentration supérieure à 50% en poids.

3. Procédé selon les revendications 1 et 2, caractérisé en ce que les catalyseurs possèdent un rapport SiO₂/Al₂O₃ supérieur à 40.

4. Procédé selon les revendications 1 à 3, caractérisé en ce que les catalyseurs possèdent un rapport SiO₂/Al₂O₃ supérieur à 100.

5. Procédé selon les revendications 1 à 4, caractérisé en ce que le catalyseur de H-Mordénite-zéolithe contient moins de 0,1% en poids de Na₂O.
